# EUROPEAN PATENT SPECIFICATION

(11) **EP 2 310 522 B1**
(45) Date of publication and mention of the grant of the patent: **20.03.2013**
(21) Application number: 09767581.3
(22) Date of filing: 16.06.2009
(51) Int. Cl.: C12P 19/34, C12Q 1/68

(54) **REAL TIME POLYMERASE CHAIN REACTION PROCESS USING A UNIVERSAL DETECTION SYSTEM**
ECHTZEIT-POLYMERASEKETTENREAKTIONSPROZESS MIT UNIVERSELLEM NACHWEISVERFAHREN
PROCÉDÉ DE RÉACTION EN CHAÎNE PAR LA POLYMÉRASE EN TEMPS RÉEL FAISANT INTERVENIR UN SYSTÈME DE DÉTECTION UNIVERSEL

(30) Priority: 17.06.2008 US 73198
(43) Date of publication of application: 20.04.2011
(73) Proprietor: Sigma-Aldrich Co. LLC, St Louis MO 63103 (US)
(72) Inventor: KREADER, Carol, St. Louis MO 63122 (US); WARD, Brian, St. Louis MO 63119 (US)
(74) Representative: Harrison, Susan Joan
(86) International application number: PCT/US2009/047473
(87) International publication number: WO 2009/155271

(56) References cited:
- WO-A1-2008/076948
- WO-A2-2007/019492
- US-A1- 2006 035 225
- US-A1- 2006 094 036
- US-A1- 2007 105 138
- US-A1- 2007 292 865
- HOSONO NAOYA ET AL: "Multiplex PCR-based real-time invader assay (mPCR-RETINA): A novel SNP-based method for detecting allellic asymmetries within copy number variation regions", HUMAN MUTATION, vol. 29, no. 1, January 2008 (2008-01), pages 182-189, XP000002658108, ISSN: 1059-7794
- OLIVIER MICHAEL: "The Invader assay for SNP genotyping", MUTATION RESEARCH, ELSEVIER, AMSTERDAM, NL, vol. 573, no. 1-2, 3 June 2005 (2005-06-03), pages 103-110, XP002536346, ISSN: 0027-5107, DOI: 10.1016/J.MRFMMM.2004.08.016

## Description

### FIELD OF THE INVENTION

The present invention relates to processes for detecting the amplification of a target nucleic acid. The process comprises a polymerase chain reaction method in which amplification of the target nucleic acid is detected in real time using a universal detection system.

### BACKGROUND OF THE INVENTION

Numerous methods are known in the art for the detection and quantification of a specific nucleic acid sequence. Polymerase chain reaction (PCR) technologies exponentially amplify the target nucleic acid and are some of the most sensitive methods known. Additionally, real time PCR methods in which the product is quantified after each round of amplification are much more convenient and accurate than traditional PCR methods in which the amplified product is detected at the end of the reaction.

Real time PCR typically detects the amplified product during the reaction by using a fluorescent reporter. The reporter may be a fluorescent dye that binds double-stranded nucleic acids or a sequence-specific hybridization probe that relies upon fluorescence resonance energy transfer (FRET) for quantitation. While the sequence-specific hybridization probes are generally more specific and accurate than a general dye, they are also more expensive. Furthermore, each target nucleic acid requires its own sequence-specific fluorescent hybridization probe, further increasing the cost of real time PCR.

What is needed, therefore, is a real time PCR detection system that utilizes a universal detection module that includes additional specificity afforded by probe-based detection. Ideally, a universal detection module would comprise a detection system that would interact with a plurality of unlabeled probes having sequence complementarity to the target nucleic acid. Thus, one universal detection module could be used to detect a plurality of target nucleic acids. Such a system would optimize the specificity of detection, but would minimize the expense of real time PCR.

WO 2007/019492 describes nucleic acid, flap-mediated, sequential amplification methods which permit detection of a nucleic acid in a nucleic acid sample. Both linear and exponential amplification methods are provided for.

Hosono et al., Human Mutation (2008) 29(1):182-189 describe the development of a real-time Invader assay combined with multiplex PCR (mPCR-RETINA) to measure the allelic ratio in copy number variation regions of a genome.

### SUMMARY OF THE INVENTION

One aspect of the present invention encompasses a real time polymerase chain reaction (PCR) process for detecting amplification of a target nucleic acid. The process comprises repeated cycles of PCR, during which each cycle comprises duplicating the target nucleic acid. Each cycle also comprises hybridizing an unlabeled probe to a region of the amplified target nucleic acid. The unlabeled probe comprises at least one portion that is complementary to the target nucleic acid and at least one portion that is not complementary to the target nucleic acid. Hybridization between the unlabeled probe and the target nucleic acid forms a structure or a sequence that is a target for cleavage. Each cycle further comprises cleaving the unlabeled probe to release a fragment. Lastly, each cycle comprises detecting the released fragment using a universal detection module the universal detection module comprising a region that is complementary to a portion of the released fragment, and the universal detection molecule comprising a hairpin structure. The detection module generates a signal in the presence of the released fragment, such that a change in the signal relative to background indicates amplification of the target nucleic acid.

Also described is a kit for detecting amplification of a target nucleic acid during a real time quantitative polymerase chain reaction process. The kit comprises at least one detection module comprising a pair of fluorescence resonance energy transfer (FRET) interactive moieties and a region that is complementary to a fragment of an unlabeled probe. The kit further comprises a thermostable DNA polymerase and a thermostable endonuclease.

Other aspects and features of the invention are described in more detail herein.

### DESCRIPTION OF THE FIGURES

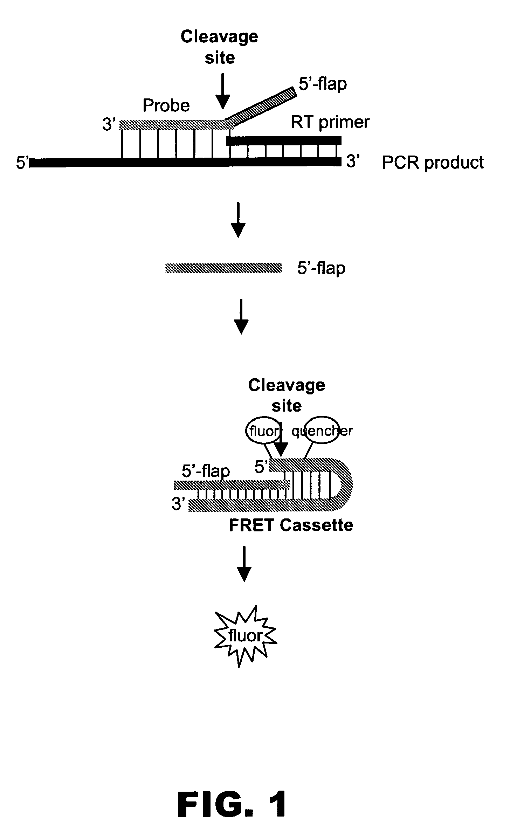

**Figure 1** diagrams a universal detection system utilizing a structure-specific endonuclease that cleaves a duplex structure comprising a 5' flap. The complementary 3' portion of an unlabeled probe hybridizes with the target nucleic acid such that it overlaps with the 3' end of a hybridized primer or probe, while the non-complementary 5' portion of the unlabeled probe remains single stranded. A structure-specific endonuclease cleaves within the duplex portion of the unlabeled probe and releases a fragment. The released fragment hybridizes with a FRET detection cassette such that a second cleavage structure is formed. The structure-specific endonuclease cleaves within the hybridized detection cassette, thereby separating the fluorescent and quencher moieties and generating a change in the fluorescence signal.

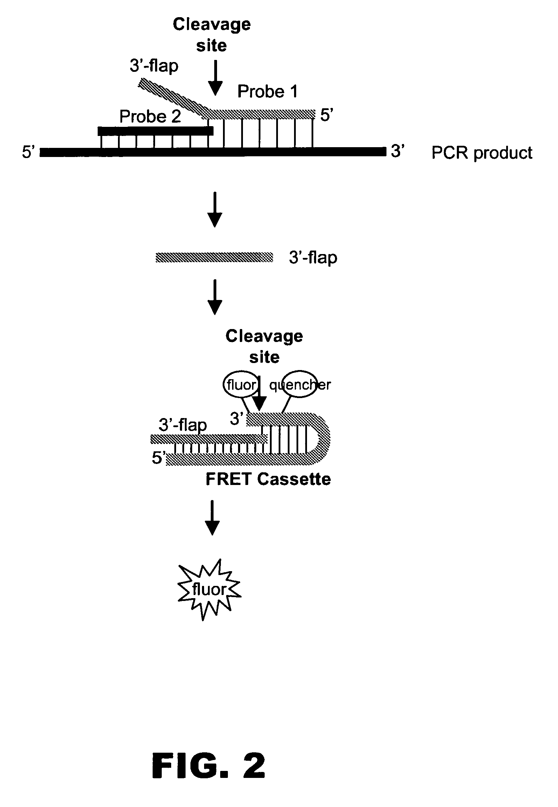

**Figure 2** diagrams a universal detection system utilizing a structure-specific endonuclease that cleaves a duplex structure comprising a 3' flap. The unlabeled probe comprises two oligonucleotides: probe 1 and probe 2. The complementary 5' portion of probe 1 hybridizes with the target nucleic acid such that it overlaps with the 5' end of hybridized probe 2, while the non-complementary 3' portion of probe 1 remains single stranded. A structure-specific endonuclease cleaves within the duplex portion of probe 1 and releases a fragment. The released fragment hybridizes with a FRET detection cassette such that a second cleavage structure is formed. The structure-specific endonuclease cleaves within the hybridized detection cassette, thereby separating the fluorescent and quencher moieties and generating a change in the fluorescence signal.

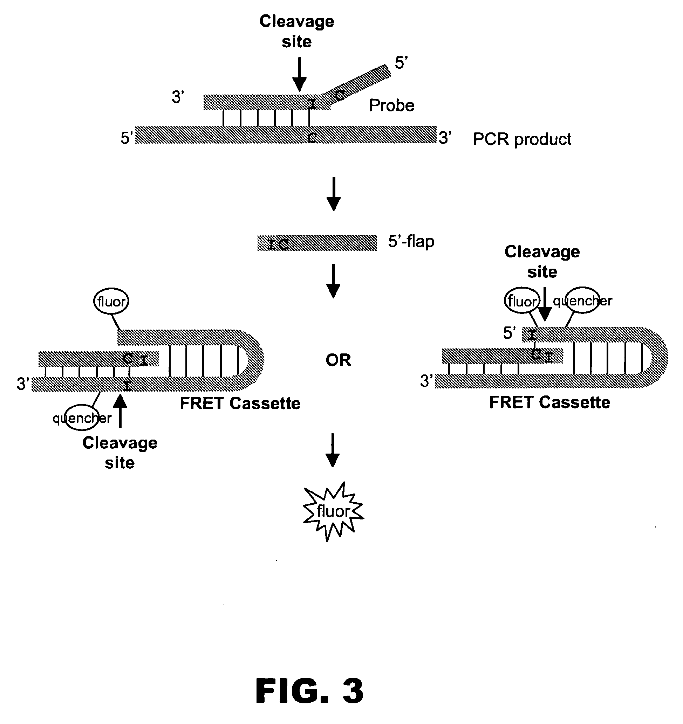

**Figure 3** diagrams a universal detection system utilizing a sequence-specific endonuclease. Endonuclease V recognizes an inosine residue in a nucleic acid and cleaves at the 3' side of the inosine residue. The unlabeled probe comprises an inosine residue. The complementary 3' portion of the unlabeled probe hybridizes with the target nucleic acid, while the non-complementary 5' portion of the unlabeled probe remains single stranded. The endonuclease cleaves 3' of the inosine residue in the unlabeled probe and releases a fragment. The released fragment hybridizes with a FRET detection cassette comprising an inosine residue. The fluorescent and quencher moieties may be at the two ends of the cassette (left), or the fluorescent and quencher moieties may be near the same end of the cassette (right). The endonuclease cleaves 3' of the inosine residue in the detection cassette, thereby separating the fluorescent and quencher moieties and generating a change in the fluorescence signal.

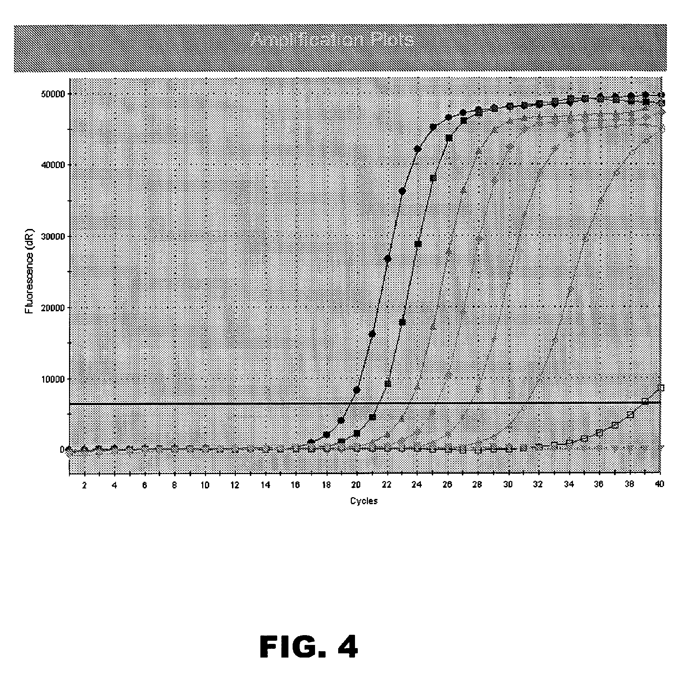

**Figure 4** presents amplification plots for serial dilutions of standard RNA using real time quantitative PCR with a structure-specific cleavage detection system. The fluorescence is plotted against the cycle number for initial copy numbers of 300,000, 75,000, 18,800, 4,700, 1,180, 295, or 74, from left to right, respectively, and a no template control.

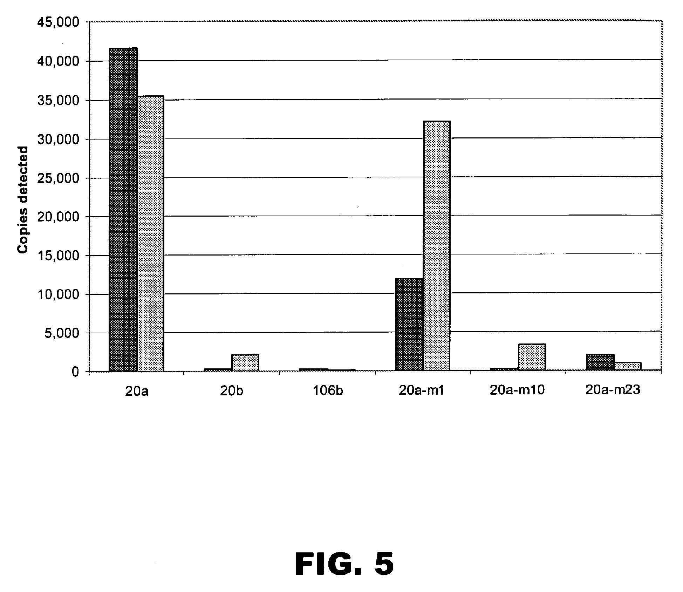

**Figure 5** is a bar graph illustrating the specificity of the reaction in which a wild type or a mutant microRNA was amplified, using primers specific for hsa-miR20a. The number of copies detected via real time quantitative PCR using a structure-specific cleavage detection system (black) or real time quantitative PCR using TAQMAN^{®} probes (gray) are plotted for each target (hsa-miR20a, hsa-miR20b, hsa-miR106b, mutant hsa-miR20a-m1, mutant hsa-miR20a-m10, and mutant hsa-miR20am23).

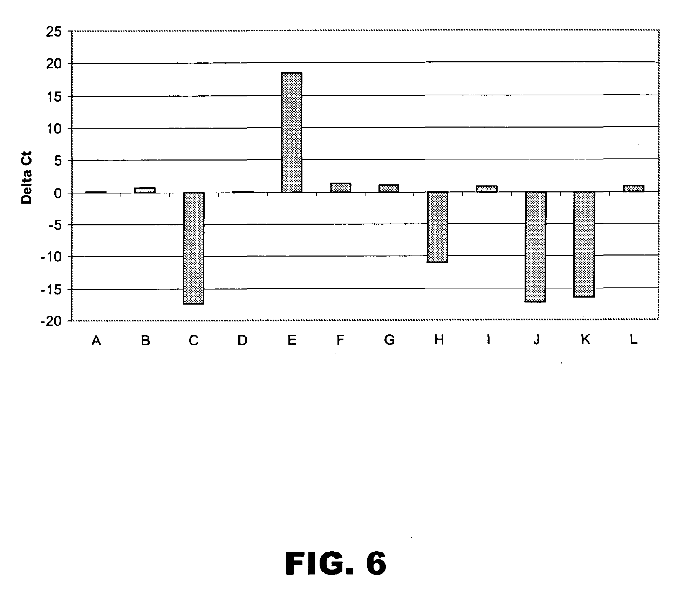

**Figure 6** illustrates the detection of single nucleotide polymorphisms using real time quantitative PCR with a structure-specific cleavage detection system. Presented are the delta Ct values for different preparations of DNA. A and B represent commercially purchased preparations of DNA; C represents DNA purified from HeLa cells; D represents DNA purified from HEK293 cells; E represents DNA purified from THP1 cells; F represents DNA purified from Wi38 cells; G and H represent DNA purified from two blood samples; and I-L represent DNA extracted from four blood samples.

### DETAILED DESCRIPTION OF THE INVENTION

It has been discovered that amplification of a target nucleic acid may be detected during a real time quantitative PCR process using a universal detection system. The process utilizes an unlabeled probe to detect the amplified target nucleic acid. The unlabeled probe comprises at least one portion that is complementary to the target nucleic acid and at least one portion that is not complementary to the target nucleic acid. Hybridization between the unlabeled probe and amplified target nucleic acid creates a specific structure or a specific sequence that is a target for cleavage. A structure-specific or a sequence-specific endonuclease cleaves within the hybridized unlabeled probe and releases a fragment. The released fragment is detected by a universal detection module comprising a detection means. Hybridization of the released fragment with the universal detection module generates another specific structure or sequence whose cleavage generates a change in the signal relative to background. The change in the signal indicates the presence of the released fragment, which in turn indicates the synthesis of the target nucleic acid. The universal detection system of the invention, as illustrated in the examples, is efficient, specific, and sensitive.

### (I) Real Time Quantitative PCR Process Using a Universal Detection System to Detect Amplification of a Target Nucleic Acid.

One aspect of the present invention provides a real time polymerase chain reaction process with a universal detection system that may be used to detect amplification of a target nucleic acid. During each cycle of the process the target nucleic acid may be duplicated and detected. Detection of the amplified target nucleic acid comprises hybridization between an unlabeled probe and the target nucleic acid, cleavage of the unlabeled probe to release a fragment, hybridization between the released fragment and the universal detection module, cleavage of the universal detection module to produce a quantifiable moiety, whereby a signal from the quantifiable moiety is generated by the detection means of the detection module if the target nucleic acid has been amplified.

### (a) amplifying the target nucleic acid

Amplification of a target nucleic acid refers to the exponential production of additional copies of a nucleic acid sequence by a method catalyzed by an enzyme. The method is generally performed using polymerase chain reaction (PCR) technologies. A variety of amplification methods are known in the art and are described, *inter alia,* in U.S. Pat. Nos. 4,683,195 and 4,683,202 and in PCR Protocols: A Guide to Methods and Applications, ed. Innis et al., Academic Press, San Diego, 1990. Essential components of the PCR technique include the template nucleic acid, at least one amplification primer, a thermostable DNA polymerase, and deoxynucletide triphosphates. PCR comprises repeated cycles of denaturation of the template nucleic acid, hybridization of the amplification primers with the target nucleic acid, and extension of the primers by the DNA polymerase.

### (i) amplification primers

Amplification primers provide initiation sites for nucleic acid synthesis. A primer is an oligonucleotide comprising a sequence that is complementary to a region of one strand of the target nucleic acid and provides a site (3' hydroxyl groups) for the initiation of synthesis of a complementary strand. Typically, a pair of amplification primers is used to amplify a region of the target nucleic acid. Each primer is complementary to either the 5' end or the 3' end of the region to be amplified.

The 3' portion of an amplification primer will generally have complete or nearly complete complementarity to the target nucleic acid. It is well known in the art that it is difficult for a DNA polymerase to synthesize a DNA strand when there is a mismatch between the template strand and the 3' end of the primer. In particular, the 3' terminal nucleotide of an amplification primer will generally be an exact match. The 5' portion of an amplification primer may have partial complementarity or no complementarity to the target nucleic acid. In general, each primer will have minimal predicted secondary structure, will not hybridize with the other primer (especially at the 3' end), and will hybridize with only the intended target nucleic acid and at only one site in that nucleic acid.

The length of the amplification primers may range from about 10 to about 40 nucleotides, preferably from about 12 to about 30 nucleotides, and even more preferably from about 14 to about 25 nucleotides. Each primer of a primer pair may have a different length. The melting temperature (or Tₘ) of one primer in a primer pair will generally be within several degrees of the other primer of the pair. Methods for estimating the Tₘ value of an oligonucleotide primer are well known in the art (e.g., see the "Definitions"). The Tₘ of a pair of primers may range from about 50°C to about 80°C, and more preferably from 60°C to about 72°C. The number of nucleotides in the target nucleic acid between the binding sites of the two primers can and will vary, depending upon a variety of factors including the sequence of the nucleic acid to be amplified, the type of nucleic acid to be amplified, the desired size of the amplicon, and so forth.

### (ii) thermostable DNA polymerase

A thermostable DNA polymerase is a polymerase that retains catalytic activity at an elevated temperature. Suitable thermostable DNA polymerases are substantially stable at elevated temperatures and efficiently catalyze amplification of a target nucleic acid in a thermal cycling process. For example, suitable thermostable DNA polymerases generally have an optimum temperature range from about 40°C to 110°C, and more typically, from about 50°C to about 95°C.

The thermostable DNA polymerase may be obtained from a variety of sources. Representative heat-stable polymerases include the DNA polymerases isolated from the thermophilic bacteria *Thermus flavus, Thermus ruber, Thermus thermophilus, Bacillus stearothermophilus, Thermus aquaticus, Thermus lacteus, Thermus rubens,* and *Methanothermus fervidus.* Thermostable DNA polymerases isolated from the thermophilic archaebacteria include, for example, *Pyrococcus furiosus, Pyrococcus woesii, Sulfolobus solfataricus, Sulfolobus acidocaldarius, Thermoplasma acidophilum, Methanobacterium thermoautotrophicum* and *Desulfurococcus mobilis.*

In one embodiment, the thermostable DNA polymerase may be from *Thermus flavus* (Tfl). In another embodiment, the thermostable DNA polymerase may be from *Thermus thermophilus* (Tth). In an alternate embodiment, the thermostable DNA polymerase may be from *Thermococcus litoralis* (Tli). In still another embodiment, the thermostable DNA polymerase may be from *Pyrococcus furiosus* (Pfu). In a preferred embodiment, the thermostable DNA polymerase may be from *Thermus aquaticus* (Taq). Thermostable DNA polymerases are commercially available from a variety of sources. The thermostable DNA polymerase may be a wild-type enzyme or a modified enzyme. In some embodiments, the thermostable DNA polymerase may have 5' and/or 3' exonuclease activity, and in other embodiments the thermostable DNA polymerase may lack 5' and/or 3' exonuclease activity.

### (iii) target nucleic acid

The target nucleic acid that is detected by the method of the invention may be a single-stranded or a double-stranded molecule. In particular, the target nucleic acid may be a RNA molecule, a DNA molecule, or a hybrid RNA-DNA molecule. Non-limiting examples of suitable DNA molecules include complementary DNA (cDNA), eukaryotic nuclear DNA, eubacterial genomic DNA, archaeal genomic DNA, viral DNA, mitochondrial DNA, chloroplast DNA, and kinetoplast DNA. The target DNA may have single nucleotide polymorphisms. Suitable RNA species include, but are not limited to, messenger RNA (mRNA), microRNA (miRNA), short interfering RNA (siRNA), repeat-associated siRNA (rasiRNA), transacting siRNA (tasiRNA), Piwi-interacting RNA (piRNA), 21 U-RNA, small nuclear RNA (snRNA), small nucleolar RNA (snoRNA), 23S/28S (16S/18S) ribosomal RNA (rRNA), 5.8S rRNA, 5S rRNA, and transfer RNA (tRNA). To detect an RNA molecule by PCR, it is generally first converted to a cDNA via reverse transcriptase-mediated reverse transcription (RT). The RT also comprises an RT primer, which may be one of the amplification primers. Examples of a suitable reverse transcriptase include wild-type, recombinant, or modified recombinant version of the reverse transcriptase from the Moloney murine leukemia virus (M-MLV) or the reverse transcriptase from the avian myeloblastosis virus (AMV).

The target nucleic acid may be derived from a natural source or it may be synthetically produced. Suitable sources of nucleic acid include eukaryotes, eubacteria, archaea, and viruses. Non-limiting examples of suitable eukaryotes include humans, mice, mammals, vertebrates, invertebrates, plants, fungi, yeast, and protozoa. The target nucleic acid may be derived from a cell, a tissue from a multicellular organism, a whole organism, a body fluid, or any other nucleic acid-containing preparation. Non-limiting examples of a suitable body fluid include blood, serum, saliva, cerebrospinal fluid, pleural fluid, lymphatic fluid, milk, sputum, semen, and urine.

### (b) hybridizing an unlabeled probe to the amplified target nucleic acid

Detection of the amplified target nucleic acid relies upon hybridization of a portion of an unlabeled probe to a region of the amplified target nucleic acid. Another portion of the unlabeled probe does not hybridize with the target nucleic acid and remains single stranded. Hybridization between the unlabeled probe and the amplified target nucleic acid generates a specific structure or a specific sequence that is a target for cleavage. The cleavage structure or sequence is recognized by a structure-specific or sequence-specific endonuclease and the unlabeled probe is cleaved, thereby releasing a fragment. The released fragment is then detected by a universal detection module.

The unlabeled probe may be a single oligonucleotide or it may be more than one oligonucleotide. In general, the unlabeled probe comprises at least one portion that is complementary to the target nucleic acid. The portion of the unlabeled probe that is complementary to the target nucleic acid may be at the 3' end of the unlabeled probe, the 5' end of the unlabeled probe, the region between the 3' and 5' ends, or a combination thereof. In embodiments in which the portion of the unlabeled probe that hybridizes with the target nucleic acid includes the 3' end of the unlabeled probe, the 3' end of the unlabeled probe may lack a free 3' hydroxyl group, such that extension of the probe is prevented. For example, the 3' terminal end of the unlabeled probe may comprise a dideoxynucleotide, an amino group, a phosphate group, or another group that is not easily removed. The unlabeled probe also comprises at least one portion that is not complementary to the target nucleic acid (but is complementary to a portion of a universal detection module).

The length of the unlabeled probe can and will vary depending upon the application. The probe may range from about 10 nucleotides to about 100 nucleotides, and more preferably from about 20 nucleotides to about 40 nucleotides. The portion (or portions) of the probe having complementarity to the target nucleic acid may range from about 8 nucleotides to about 25 nucleotides, and more preferably from about 10 nucleotides to about 18 nucleotides. Typically, the probe will not hybridize with either of the amplification primers and will hybridize with only one site in the target nucleic acid.

In some embodiments, hybridization between the unlabeled probe and the target nucleic acid may generate a cleavage structure, i.e., a region having secondary structure. Formation of the secondary structure does not require the synthetic activity of a DNA polymerase, but rather the secondary structure is formed by hybridization between the unlabeled probe and the target nucleic acid. The region with secondary structure may be recognized by a structure-specific endonuclease, which cleaves within the unlabeled probe region of the structure and releases a fragment.

In one embodiment, the cleavage structure may comprise a duplex region in which the unlabeled probe comprises a single-stranded 5' flap region. For this, the unlabeled probe may comprise a 3' portion that is complementary with the target nucleic acid and a 5' portion that is not complementary with the target nucleic acid. Hybridization between the complementary 3' portion of the unlabeled probe and the target nucleic acid may form a duplex structure in which the hybridized region of the unlabeled probe abuts or overlaps with the 3' end of another duplex region, and with the non-complementary 5' portion of the unlabeled probe remaining single stranded, thereby forming the 5' flap (see Figure 1). The other duplex region of the structure may comprise an amplification primer or it may comprise a second unlabeled probe. Cleavage of the unlabeled probe releases a fragment comprising the 5' flap.

In another embodiment, the cleavage structure may comprise a duplex region having a 3' flap. For this, the unlabeled probe may comprise two separate oligonucleotides: probe 1 and probe 2. Probe 1 may comprise a 5' portion that is complementary with the target nucleic acid and a 3' portion that has no complementarity with the target nucleic acid. Probe 2 may be complementary to a region of the target nucleic acid that is 3' to the region recognized by probe 1. Hybridization of the complementary 5' portion of probe 1 with the target nucleic acid may form a duplex structure in which the hybridized region of probe 1 abuts or overlaps with the 5' end of hybridized probe 2, with the non-complementary 3' portion of probe 1 remaining single stranded, thereby forming the 3' flap. Cleavage of hybridized probe 1 releases a fragment comprising a 3' flap (see Figure 2).

In still another embodiment, hybridization between the unlabeled probe and the target nucleic acid may form a cleavage structure comprising a single-stranded loop. For this, complementary 5' and 3' regions of the unlabeled probe hybridize with the target nucleic acid, and a non-complementary internal region forms a single-stranded loop. Cleavage of the structure releases a fragment of the unlabeled probe. In iterations of this embodiment, the single-stranded loop region may comprise complementary sequences such that it base pairs with itself to form a stem, a hairpin, a stem-loop, a knot, and the like.

In other embodiments, hybridization between the unlabeled probe and the target nucleic acid may generate a region comprising a specific nucleotide or specific sequence of nucleotides that is a target for cleavage (e.g., see Figure 3). After hybridization of the unlabeled probe, a sequence-specific endonuclease may recognize the specific nucleotide or the specific sequence of nucleotides and cleave the unlabeled probe within or near the specific nucleotide or specific sequence of nucleotides to release a fragment.

The location of the region of the target nucleic acid that hybridizes with the unlabeled probe may vary. In some embodiments, the unlabeled probe may hybridize with a region of the target nucleic acid that is adjacent to one of the amplification primers. The duplex region of the hybridized unlabeled probe may abut or overlap with one of the hybridized amplification primers. The amount of overlap between the hybridized unlabeled probe and the hybridized amplification primer may vary. There may be an overlap of one nucleotide. Alternatively, the amount of overlap may be two, three, four, or five nucleotides. In a preferred embodiment, the 3' portion of the unlabeled probe may hybridize with the target nucleic acid such that the hybridized portion of the unlabeled probe overlaps with the 3' end of the hybridized upstream primer, while the 5' portion of the unlabeled probe remains single-stranded. In other embodiments, the unlabeled probe may hybridize with a region of the target nucleic acid that lies between the hybridization sites of the two amplification primers.

The melting temperature (Tₘ) of the hybridized region of the unlabeled probe may vary. In one embodiment, the Tₘ of the unlabeled probe may be below the Tₘ of the pair of amplification primers. The Tₘ of the probe may be about 20°C, about 15°C, about 10°C, or about 5°C below the Tₘ of the amplification primers. In another embodiment, the Tₘ of the unlabeled probe may be about the same as the Tₘ of the amplification primers. In still another embodiment, the Tₘ of the unlabeled probe may be above the Tₘ of the amplification primers. The Tₘ of the unlabeled probe may be about 5°C, about 10°C, about 15°C, or about 20°C above the Tₘ of the amplification primers.

Depending upon the melting temperatures of the amplification primers and the unlabeled probe, the cycling parameters of the PCR process may be adjusted to provide separate temperature steps for cleavage and for amplification. In one embodiment, the Tₘ of the unlabeled probe may be about 50°C and the Tₘ of the amplification primers may be about 60°C. Thus, a 50°C step may be introduced between the denaturation step and the annealing/elongation step of a cycle (e.g., Examples 1 and 2). One skilled in the art will appreciate that the primers may also anneal at the lower temperature and elongation may also proceed at the lower temperature. In other embodiments, the Tₘ of the unlabeled probe may be about the same as or higher than the Tₘ of the amplification primers. In these embodiments, PCR cycles comprising a denaturation step and a single annealing/elongation/cleavage step may suffice (e.g., Example 4).

### (c) cleaving the unlabeled probe

Hybridization between the unlabeled probe and the amplified target nucleic acid generates a specific structure or a specific sequence that may be recognized and cleaved by a thermostable endonuclease. A thermostable endonuclease is an enzyme that substantially retains catalytic activity at an elevated temperature. The elevated temperature includes, but is not limited to, about 45°C, about 50°C, about 55°C, about 60°C, about 65°C, about 70°C, about 75°C, about 80°C, about 85°C, or about 90°C. The thermostable endonuclease may be a wild-type enzyme or a modified enzyme obtained from thermophilic organisms such as *Acidianus ambivalens, Acidianus brierlyi, Aeropyrum pernix, Archaeoglobus fulgidus, Archaeaglobus profundus, Archaeaglobus veneficus, Desulfurococcus amylolyticus, Desulfurococcus mobilis, Methanobacterium thermoautotrophicum, Methanococcus igneus, Methanococcus jannaschii, Methanopyrus kandleri, Pyrobaculum aerophilum, Pyrococcus furiosus, Pyrococcus horikoshii, Pyrococcus woesei, Pyrodictium brockii, Sulfolobus solfataricus, Thermus aquaticus, Thermus flavus, Thermus thermophilus, Thermococcus gorgonarius, Thermococcus litoralis,* and *Thermococcus zilligii.*

In some embodiments, the thermostable endonuclease may be a structure-specific endonuclease. Generally speaking, a structure-specific endonuclease is an enzyme that recognizes and cleaves at or near a specific secondary structure, rather than a specific sequence of nucleotides. In the context of this invention, a structure-specific endonuclease recognizes a specific secondary structure and cleaves within the unlabeled probe region of the secondary structure to release a fragment. Examples of secondary structures that may be recognized and cleaved by a structure-specific endonuclease include a duplex structure comprising a flap, an overlapping duplex structure comprising a flap, a duplex structure comprising a loop (e.g., an internal loop, a bulge loop, or a bubble loop), or a duplex structure comprising a stem (e.g., a stem-loop or a hairpin). The structure-specific endonuclease may be a flap endonuclease, a 5' flap endonuclease, a 3' flap endonuclease, a loop endonuclease, a hairpin endonuclease, a DNA polymerase, or a combination thereof. Without departing from the scope of the invention, the structure-specific endonuclease may also include an enzyme involved in DNA repair, DNA remodeling, or RNA processing that cleaves one strand of a secondary structure.

In a preferred embodiment, the structure-specific endonuclease may be a thermostable FEN-1 endonuclease. Thermostable FEN-1 endonucleases have been isolated from thermophilic organisms such as *Acidianus ambivalens, Acidianus brierlyi, Aeropyrum pemix, Archaeaglobus profundus, Archaeaglobus veneficus, Desulfurococcus amylolyticus, Desulfurococcus mobilis, Methanococcus igneus, Methanopyrus kandleri, Pyrobaculum aerophilum, Pyrococcus horikoshii, Pyrodictium brockii, Sulfolobus solfataricus, Thermococcus gorgonarius, Thermococcus litoralis,* and *Thermococcus zilligii.* FEN-1 endonucleases have been described in U.S. Pat. Nos. 5,846,717, 5,985,557; 5,994,069; 6,001,567; 6,090,543; 6,090,606; 6,348,314; 6,458,535; 7,045,289; 7,122,364; 7,150,982; PCT Appl. Nos. WO 97/27214; WO 98/42873; Lyamichev et al., Nature Biotechnol, 17:292-296 (1999); and Hall et al., Proc Natl Acad Sci, USA, 97:8272-8277 (2000).

In alternate embodiments, the thermostable endonuclease may be a sequence-specific endonuclease. Sequence-specific endonucleases useful in this invention include those that recognize a specific sequence or specific nucleotide in a nucleic acid duplex, cleave one strand of the duplex, i.e., the unlabeled probe strand, and release a fragment. An example of a suitable endonuclease is provided by endonuclease V, which recognizes an inosine residue in a nucleic acid and cleaves on its 3' side (see Figure 3). Other suitable endonucleases include those that recognize and cleave abasic sites. Those skilled of skill in the art will appreciate that the inosine residue or the abasic site may be in the hybridized or unhybridized portion of the unlabeled probe, that the endonuclease must be thermophilic, and that any endonuclease with activity on single-stranded DNA would require engineering to remove that activity to avoid cleaving the FRET cassette in the absence of released fragment.

### (d) detecting the released fragment

Cleavage of the unlabeled probe in the cleavage structure (or sequence) releases a fragment, which is then detected by a universal detection module. The released fragment hybridizes with the detection module and forms a secondary cleavage structure (or sequence). A structure-specific (or sequence-specific) endonuclease recognizes and cleaves the detection module, whereby the detection means generates a signal. Detection of increasing amounts of the released fragment indicates that the target nucleic acid has been amplified. Because the released fragment has little or no sequence complementarity to the target nucleic acid, it is essentially target-independent. Thus, more than one unlabeled probe may comprise the same "released fragment" portion, and a single detection module may detect the released fragment from more than one unlabeled probe. Thus, the detection module of the invention is a universal detection module in that it may detect the amplification of a plurality of target nucleic acids.

The detection module is an oligonucleotide that has a region with complementarity to a portion of the released fragment. The location of the region having complementarity to the released fragment can and will vary, depending upon the design of the module. In one embodiment, a 3' region of the detection module may have complementarity to the released fragment. In another embodiment, a 5' region of the detection module may have complementarity to the released fragment. The detection module also comprises a hairpin structure, which has a double-stranded stem region and a single-stranded loop region. The hairpin structure may be within a 5' region or a 3' region of the detection module.

The length of the oligonucleotide detection module can and will vary depending upon the design of the detection module and the detection strategy. In general, the detection module may range from about 20 nucleotides to about 100 nucleotides. The length of the region having complementarity to the released fragment can and will vary depending upon, among other parameters, the desired melting temperature of the hybrid. In general, the region having complementarity to the released fragment may range from about 6 nucleotides to about 20 nucleotides. The melting temperature of the hybridized region of the detection module may vary. Typically, the melting temperature of the detection module will be about the same as that of the unlabeled probe, such that hybridization of the unlabeled probe and hybridization of the released fragment will occur concurrently during the polymerase chain reaction process.

The detection module also comprises a detection means. The detection means of the detection module typically comprises at least one fluorescent moiety, which may be used to generate a detectable signal. The signal may be a change in fluorescence emission or a change in fluorescence polarization. In some embodiments, the detection module may comprise a pair of fluorescence resonance energy transfer (FRET) interactive moieties. The FRET interactive moieties may comprise two different fluorescent dyes. Alternatively, the FRET interactive moieties may comprise a fluorescent dye and a quencher dye. A variety of different fluorescent and quenching moieties, well known in the art, may be included in the detection module. The relative locations of the moieties in the detection module can and will vary depending upon the design of the detection module. In one embodiment, a FRET pair may be positioned such that one is at or near the 5' end of the detection module and the other is within the 5' region of the detection module. In another embodiment, a FRET pair may be positioned such that one is at or near the 3' end of the detection module and the other is within the 3' region of the detection module. In still another embodiment, a FRET pair may be positioned such that one is near the 5' end or within the 5' region and the other is near the 3' end or within the 3' region of the detection module.

Hybridization between the released fragment and the detection module forms a specific structure or sequence that is a target for cleavage. A structure-specific endonuclease may recognize the cleavage structure in the hybridized detection module and cleave the detection module, whereby the detection means generates a signal (see Figures 1 and 2). Alternatively, a sequence-specific endonuclease may recognize the specific nucleotide or sequence of nucleotides in the hybridized detection module and cleave the detection module, whereby the detection means generates a signal (see Figure 3). Structure-specific and sequence-specific endonucleases were described above in section (I)(c). A change in the signal relative to background indicates the presence of the released fragment, which in turn indicates amplification of the target nucleic acid. The signal produced by the detection means of the detection module may be increased relative to background. Alternatively, the signal produced by the detection means may be decreased relative to background.

In a preferred embodiment, the detection module may comprise a 3' region that hybridizes with the released fragment, a 5' region that forms a hairpin loop structure, and a pair of FRET interactive moieties (e.g., one is located at the 5' end of the detection module and the other is located within the 5' region of the detection module). Upon hybridization of the released fragment, the hybridized fragment may overlap with a region in the 5' region of the detection module such that a secondary cleavage structure is formed. A structure-specific endonuclease may recognize the secondary structure and cleave the detection module, thereby separating the FRET interactive moieties and generating a fluorescent signal.

In a further embodiment, the process of the invention may be utilized to detect the amplification of more than one target nucleic acid during a single polymerase chain reaction, as demonstrated in Example 5. For this, each target nucleic acid will generally have a unique set of amplification primers and a unique unlabeled probe. Each of these unlabeled probes will have complementarity to a specific target nucleic acid, as well as complementarity to a different detection module. The number of detection modules can and will vary, depending upon the number of target nucleic acids to be detected. In general, each detection module will hybridize with a specific released fragment and will have a different fluorophore or a different combination of FRET pairs.

### (II) Kits for Detecting Amplification of a Target Nucleic Acid During Real Time Quantitative PCR Using a Universal Detection Module.

Also described herein are kits for detecting amplification of a target nucleic acid using the method of the invention. A kit comprises at least one detection module, as described above in section (I)(d); a thermostable DNA polymerase, as described above in section (I)(a)(ii); and a thermostable endonuclease, as described above in section (I)(c). Target nucleic acids that may be detected with a kit were detailed in above section (I)(a)(iii).

A kit may further comprise a buffering agent, a divalent cation, a monovalent cation, a detergent, and a mixture of deoxynucleotide triphosphates (dNTPs). Suitable buffering agents include those known in the art that will maintain the pH of the reaction from about 7.0 to about 9.5. Representative examples of suitable buffering agents include Tris buffers, MOPS, HEPES, Bicine, Tricine, TES, or PIPES. A suitable divalent cation for use in the method includes magnesium and/or manganese. Suitable monovalent cations include potassium, sodium, or lithium. Detergents that may be included include poly(ethylene glycol)4-nonphenyl 3-sulfopropyl ether potassium salt, 3-[(3-cholamidopropyl)dimethylammonio]-1-propanesulfonate, 3-[(3-cholamidopropyl)dimethylammonio]-2-hydroxy-1-propanesulfonate, Tween 20, and Nonidet NP40. A mixture of dNTPs typically comprises dATP, dCTP, dGTP, dTTP and/or dUTP. Other agents that may be included include glycerol and/or polyethylene glycol.

A kit may also comprise a reverse transcriptase. The reverse transcriptase may be a wild-type, recombinant, or modified recombinant version of the reverse transcriptase from the Moloney murine leukemia virus (M-MLV) or the reverse transcriptase from the avian myeloblastosis virus (AMV).

A kit may also comprise at least one unlabeled probe, which was described above in section (I)(b). A kit may also comprise at least one pair of amplification primers, as described above in section (I)(a)(i).

### DEFINITIONS

To facilitate understanding of the invention, a number of terms are defined below:

As used herein, the terms "complementary" or "complementarity" refer to the natural association of nucleic acid sequences by base pairing (i.e., 5'-A G T-3' pairs with the complimentary sequence 3'-T C A-5'). Complementarity between two single-stranded molecules may be partial, if only some of the nucleic acid pairs are complimentary, or complete, if all the base pairs are complimentary.

The term "hybridization," as used herein, refers to the process of annealing by base pairing and hydrogen bond formation between two complementary strands of nucleic acids. Hybridization and the strength of hybridization (i.e., the strength of the association between the nucleic acids) are influenced by such factors as the degree of complementary between the nucleic acids, the percent of G+C, the Tₘ of the formed hybrid, and the stringency of the conditions involved.

As used herein, the term "modified enzyme," refers to an enzyme that displays altered functional characteristics relative to the wild-type enzyme. The modifications may be engineered using recombinant DNA technologies or may be due to naturally occurring mutations.

The term "oligonucleotide," as used herein, refers to a molecule comprising two or more nucleotides. The nucleotides may be standard nucleotides (i.e., adenosine, guanosine, cytidine, thymidine, and uridine) or nucleotide analogs. A nucleotide analog refers to a nucleotide having a modified purine or pyrimidine base or a modified ribose moiety. A nucleotide analog may be a naturally occurring nucleotide (e.g., inosine) or a non-naturally occurring nucleotide. Non-limiting examples of modifications on the sugar or base moieties of a nucleotide include the addition (or removal) of acetyl groups, amino groups, carboxyl groups, carboxymethyl groups, hydroxyl groups, methyl groups, phosphoryl groups, and thiol groups, as well as the substitution of the carbon and nitrogen atoms of the bases with other atoms (e.g., 7-deaza purines). Nucleotide analogs also include dideoxy nucleotides, 2'-O-methyl nucleotides, locked nucleic acids (LNA), peptide nucleic acids (PNA), and morpholinos. The nucleotides may be linked by phosphoester, phosphothioate, phosphoramidite, or phosphorodiamidate bonds.

The term "secondary structure," as used herein, refers to the conformation of a nucleic acid molecule comprising double-stranded helices and single-stranded regions or loops. As used herein, a "hairpin structure," refers to a double-stranded helical region formed by base paring between adjacent, inverted, complementary sequences in a single strand of nucleic acids. A "stem loop" refers to a hairpin structure, further comprising a loop of unpaired bases at one end.

As used herein, the term "Tₘ" refers to the "melting temperature," which is the temperature at which a population of double-stranded nucleic acid molecules becomes half dissociated into single strands. Several equations for calculating the Tₘ of oligonucleotides are well known in the art. For example, an estimate of the Tₘ value of an oligonucleotide less than about 14 nucleotides may be calculated by the equation: Tₘ = (2 x number of A+T) + (4 x number of G+C). And an estimate of the Tₘ value of an oligonucleotide greater than about 14 nucleotides may be calculated by the equation Tₘ = 64.9 + (41 x ((number of G+C -16.4)/total number of nucleotides)) (http://tools.bio.anl.gov/bioJAVA/jsp/ExpressPrimerTool/).

The term "universal detection module," as used herein, refers to a module that may detect the amplification of a plurality of target nucleic acids in a plurality of reactions.

The term "unlabeled probe," as used herein, refers to an oligonucleotide that does not contain an atom or molecule that may be detected directly, i.e., without additional manipulations or reactants using currently available instruments (e.g., fluorescence emission, fluorescence polarization, radioactivity, magnetism, X-ray diffraction, X-ray absorption, and the like).

As used herein, the terms "upstream" or "downstream" refer to a relative position in a strand of nucleotides. Each strand of nucleotides has a 5' end and a 3' end. Relative to the position on the strand, upstream is the region towards the 5' end of the strand and downstream is the region towards the 3' end of the strand.

The term, "wild-type enzyme," as used herein refers to an enzyme isolated from a naturally occuring source and represents the most frequently observed form of the enzyme in the natural population.

### EXAMPLES

The following examples are included to demonstrate preferred embodiments of the invention. It should be appreciated by those of skill in the art that the techniques disclosed in the examples that follow represent techniques discovered by the inventors to function well in the practice of the invention. However, those of skill in the art should, in light of the present disclosure, appreciate that many changes may be made in the specific embodiments that are disclosed and still obtain a like or similar result, therefore all matter set forth in the above description and in the examples given below, shall be interpreted as illustrative and not in a limiting sense.

### Example 1. Detection of microRNA using real time quantitative PCR with a universal detection system comprising structure-specific cleavage.

The purpose of the following experiment was to determine whether a universal detection system based upon the structure-specific cleavage (SSC) system sold under the trade name INVADER^{®} by Third Wave Technologies could be used to detect PCR products during each cycle of real time quantitative PCR (qPCR). In particular, the experiment was designed to determine whether the SSC system could be used during each cycle of PCR rather than after all the cycles of PCR were completed. For this, a MIRVADER^{®} microRNA detection kit (Third Wave Technologies, Madison, WI) specific for hsa-miR-155 was used as instructed by the manufacturer except that the thermocycling parameters were modified and the final detection step was eliminated. Since the Tₘ of the unlabeled probe was lower than the Tₘ of the amplification primers, an additional step at 50°C was added between the denaturing step (95°C) and the annealing/elongation step (60°C) of each cycle.

Each reaction contained 10.5 µL of water, 2 µL of 10X reaction buffer, 2 µL of primer-probe set, 0.5 µL of enzyme mix, and 5 µL of standard or test RNA. A stock solution of standard RNA (Third Wave Technologies) was serially diluted 4-fold with tRNA diluent to generate seven reference solutions (ranging from 300,000 copies to 73 copies of miRNA per 5 µL). The test RNA comprised two different preparations of total RNA from HeLa cells, and was diluted to a concentration of about 2 ng/µL (~10 ng RNA/reaction). Each RNA preparation was tested in triplicate. No template controls containing tRNA diluent were included. The samples were incubated at: 42°C, 30 min; 95°C, 2 min; and then 40 cycles (95°C, 20 sec; 50°C, 30 sec; 60°C, 1 min).

Amplification plots for the standard RNA are presented in Figure 4. The two lowest standards were detected at later cycles than required to fit a regression line generated by the first five standards (r² ≥ 0.98), which suggests that a longer detection step may be necessary to increase sensitivity and extend the linear range of the assay. The threshold cycle (Ct) values of the highest five standards were plotted against the log of the initial copy number to generate a standard curve. The efficiency of PCR was calculated from slope of the standard curve; the efficiency of about 100% indicates that the products were doubled during each cycle. The standard curve was also used to determine the number of copies of hsa-miR-155 in each test RNA sample.

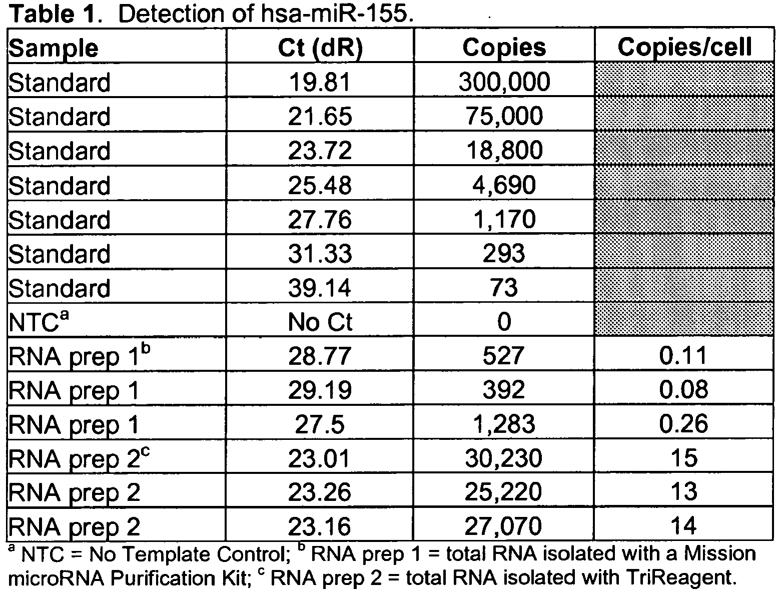

Table 1 presents the Ct value and initial copy number for each sample, as well as the number of copies/cell for the two test RNA preparations. The number of copies of hsa-miR-155 per cell detected by this modified method (RNA prep 1 = 0.15; RNA prep 2 = 14) more closely resembled the number of copies/cell determined using a standard real time qPCR assay (RNA prep 1 = 0.2; RNA prep 2 = 17) than the copies/cell determined by the standard MIRVADER^{®} assay with endpoint detection (RNA prep 1 = 1.0; RNA prep 2 = 67) (data not shown). This experiment revealed that a structure-specific cleavage detection system assay may be used to detect products during each cycle of qPCR.

### Example 2. Comparison of real time qPCR with a structure-specific cleavage detection system and a conventional qPCR method.

Several performance parameters of the qPCR assay using the structure-specific cleavage (qPCR-SSC) detection system were compared to those of qPCR using a 5'-nuclease detection assay (i.e., TAQMAN^{®} probes; Applied Biosystems, Foster City, CA). For this, hsa-miR-16, hsa-miR-20a, hsa-miR-155, and hsa-miR-342 were detected in serial dilutions from the miRNA standards (300,000 to 73 copies) and in two different preparations of human RNA. The specificity of the detection was tested using a preparation of tomato small RNA.

The reactions for the qPCR-SSC system were set up as described in Example 1. The thermocycling parameters were modified to allow more time for the detection step during each cycle: 42°C, 30 min; 95°C, 2 min; and 40 cycles (95°C, 20 sec; 50°C, 45 sec; 60°C, 30 sec). For the TAQMAN^{®} assay (qPCR-TM), cDNA was synthesized using a TAQMAN^{®} MicroRNA Reverse Transcription (RT) Kit according to the manufacturer's instructions. Each reaction contained 4.16 µL of water, 1.5 µL of 10X RT buffer, 0.15 µL of 100 mM dNTPs, 0.19 µL of RNase inhibitor, 3 µL of RT primer, 1 µL of MULTISCRIBE^{®} RT (Applied Biosystems) and 5 µL of standard or test RNA. The reactions were incubated at 16°C, 30 min; 42°C, 30 min; 85°C, 5 min, and held at 4°C. Then the cDNA was amplified using TAQMAN^{®} 2x Universal PCR Master Mix, No AMPERASE^{®} UNG in reactions comprising 8 µL of water, 10 µL of 2X Universal PCR reaction mix, 1 µL of primer-probe mix, and 1 µL of cDNA from the RT reactions. The thermocycling parameters were: 95°C, 10 min and 40 cycles (95°C, 15 sec; 60°C, 1 min).

Standard curves were generated from amplification plots of the standards and used to calculate PCR efficiency and estimate the number of copies of miRNA in each RNA preparation. Table 2 summarizes the results. The efficiency for both assays was around 100%, and the estimated number of copies of the target miRNA in the total RNA preparations was similar between the two systems. The sensitivity, which was defined as lowest linear standard, was better for two of the four targets detected with qPCR-SSC. The specificity, which was defined as the number of copies detected in the tomato small RNA, was similar between the two systems.

**Table 2. Detection copy number of the target miRNAs in the total RNA preps of miRNAs.**

| | | | **qPCR-SSC** | **qPCR-TM** |
|---|---|---|---|---|
| **hsa-miR-16** | | | | |
| | Efficiency | | 110.5% | 98.6% |
| | | R-squared | 0.999 | 1 |
| | Sensitivity^{a} | | <73 copies | <73 copies |
| | Specificity^{b} | | 46 copies | 0 copies |
| | RNA prep 1^{c} | | 4,490 copies | 5,681 copies |
| | RNA prep 2^{d} | | 6,445 copies | 7,828 copies |
| **hsa-miR-20a** | | | | |
| | Efficiency | | 104.7% | 101.5% |
| | | R-squared | 0.999 | 1 |
| | Sensitivity | | <73 copies | <73 copies |
| | Specificity | | 1 copies | 0 copies |
| | RNA prep 1 | | 4,603 copies | 2,975 copies |
| | RNA prep 2 | | 5,277 copies | 3,796 copies |
| **hsa-miR-155** | | | | |
| | Efficiency | | 95.6% | 110.5% |
| | | R-squared | 0.996 | 0.999 |
| | Sensitivity | | <73 copies | 293 copies |
| | Specificity | | 0 copies | 24 copies |
| | RNA prep 1 | | 4,147 copies | 3,336 copies |
| | RNA prep 2 | | 6,680 copies | 4,609 copies |
| **hsa-miR-342** | | | | |
| | Efficiency | | 99.7% | 110.5% |
| | | R-squared | 0.997 | 0.999 |
| | Sensitivity | | <73 copies | 1,170 copies |
| | Specificity | | 0 copies | 0 copies |
| | RNA prep 1 | | 63 copies | 28 copies |
| | RNA prep 2 | | 112 copies | 46 copies |

| | | | | |
|---|---|---|---|---|
| ^{a} Sensitivity = lowest linear standard; ^{b} Specificity = copies detected per 10 ng of tomato RNA; ^{c} RNA prep 1 = total RNA isolated with Ambion's MIRVANA™ miRNA Isolation Kit; ^{d} RNA prep 2 = total RNA isolated with TriReagent. | | | | |

This experiment revealed that the qPCR-SSC detection system gave results that were similar to those obtained with qPCR-TM system, and that the qPCR-SSC method may be slightly more sensitive. Furthermore, the qPCR-SSC system required less hands-on time than the qPCR-TM system (i.e., 1 reaction vs. two reactions per assay).

### Example 3. Specificity of the real time qPCR-SSC system.

Since the Tₘs of the amplification primers were about 60°C, it is possible that some non-specific amplification could occur during the detection step at 50°C. Thus, to more accurately assess the specificity of the real time qPCR-SSC system, synthetic miRNAs with or without mutations were tested using primers and an unlabeled probe specific for hsa-miR-20a. The sequences of the synthetic miRNAs are presented in Table 3; hsa-miR-20b differs from hsa-miR-20a by two nucleotides, hsa-miR-106b is two nucleotides shorter than hsa-miR-20a-1 and has three nucleotide substitutions, mutant hsa-miR-20a-1 has the 5'-most nucleotide changed, mutant hsa-miR-20a-10 has a middle nucleotide changed, and mutant hsa-miR-20a-23 has the 3'-most nucleotide changed.

**Table 3. Sequences of wild type and mutant miRNAs.**

| **miRNA** | **Sequence (5'-3')*** | **SEQ ID NO:** |
|---|---|---|
| hsa-miR-20a | UAAAGUGCUUAUAGUGCAGGUAG | 1 |
| hsa-miR-20b | CAAAGUGCUCAUAGUGCAGGUAG | 2 |
| hsa-miR-106b | UAAAGUGCUGACAGUGCAGAU | 3 |
| hsa-miR-20a-1 | CAAAGUGCUUAUAGUGCAGGUAG | 4 |
| hsa-miR-20a-10 | UAAAGUGCUCAUAGUGCAGGUAG | 5 |
| hsa-miR-20a-23 | UAAAGUGCUUAUAGUGCAGGUAA | 6 |

| | | |
|---|---|---|
| * Nucleotide changes shown in BOLD. | | |

The miRNAs were synthesized, desalted, and resuspended at 100 µM in 10 mM TE (Tris-EDTA solution). These stock solutions were further diluted to give 35,000 to 40,000 copies per 5 µL. RNA standards were serially diluted 5-fold to give seven standards that ranged from 300,000 copies to 19 copies per 5 µL. qPCR-SSC and qPCR-TM assays were set up and run as described in Example 2.

Figure 5 presents the results. The specificity of the qPCR-SSC detection system was just as good or better than the qPCR-TM system. Neither system could discriminate well between a perfectly matched target and a target with a mutation in the 5' most position. These data reveal that the qPCR-SSC system has sufficient specificity for the detection of miRNAs.

### Example 4. Detection of mRNAs using the real time qPCR-SSC system with short or long probes.

The detection of messenger RNAs was also tested using the qPCR-SSC system and compared to the qPCR-TM system. The target nucleic acids were mRNAs that coded for the following human kinases: CHUK (NM_001278), IKbKb (NM_001556), IRAK2 (NM_001570), MAP3K2 (NM_006609), and TBP (NM_003194).

Standard reference human RNA (Stratagene, La Jolla, CA) was diluted in tRNA diluent to provide four input levels (100, 10, 1.0, and 0.1 ng per 2 µL). Three different preparations of total RNA from HeLa cells were used after diluting them to 0.5 µg/µL.

An INVADER PLUS^{®} Kit (Third Wave Technologies) was used for the qPCR-SSC reactions. Standard "short" unlabeled probes with Tₘs about 10°C below that of the primers were provided by the manufacturer. Additionally, longer unlabeled probes with Tₘs about the same or slightly above that of the primers were also provided by the manufacturer. It was reasoned that the longer probe would hybridize with the target sequence and form the flap structure concurrently with PCR amplification, such that a separate detection step would not be needed. Note, a long probe for CHUK and a short probe for TBP were not successful synthesized. Each qPCR-SSC reaction contained 13.5 µL of water, 2 µL of 10X reaction buffer, 2 µL of a primer-probe set, 0.5 µL of enzyme mix, and 2 µL of standard RNA or test RNA. Those with the standard "short" unlabeled probe were incubated at: 42°C, 30 min; 95°C, 2 min; and 40 cycles (95°C, 20 sec; 50°C, 45 sec; 60°C, 30 sec). Those with the "longer" unlabeled probe were incubated at: 42°C, 30 min; 95°C, 2 min; and 40 cycles (95°C, 20 sec; 60°C, 1 min). No template controls containing water, tRNA, or DNA were included.

Parallel qPCR-TM assays were performed for comparison. cDNA was synthesized using a High-Capacity cDNA Reverse Transcription kit (Applied Biosystems). Each reaction contained 12.2 µL of water, 2 µL of 10X RT buffer, 0.8 µL of 25X dNTP mix, 2 µL of 10X RT random primers, 1 µL of MULTISCRIBE^{®} RT, and 2 µL of standard RNA or test RNA. Note, the standard and test RNA solutions were 10-fold more concentrated than those used for qPCR-SSC so that equal amounts of cDNA would be tested in qPCR. The reactions were incubated at 25°C, 10 min; 37°C, 120 min; 85°C, 5 sec, and held at 4°C. The cDNA was amplified via qPCR using TAQMAN^{®} 2x Universal PCR Master Mix, No AMPERASe^{®} UNG. Each reaction contained 7 µL of water, 10 µL of 2X TAQMAN^{®} Universal PCR Master Mix, 1 µL of 20X TAQMAN^{®} Gene Expression Assay Mix, and 2 µL of cDNA. The samples were thermocycled at 95°C, 10 min and 40 cycles (95°C, 15 sec; 60°C, 1 min).

Table 4 summarizes the results. The PCR efficiency was similar between the two detection systems, except for IRAK2 in which the qPCR-SSC detection system was much less efficient. It is possible that the efficiency was lower because the (IRAK2) RT primer was very GC-rich (79%), especially at its 3'-end. All assays, except two qPCR-SSC assays with long probes, were linear down to the lowest standard tested (0.1 ng). Otherwise, results for the qPCR-SSC assays with long probes were similar to those with short probes or qPCR-TM. Therefore, qPCR-SSC will likely perform well with primers and probes that both have Tₘs of about 60°C after additional optimization.

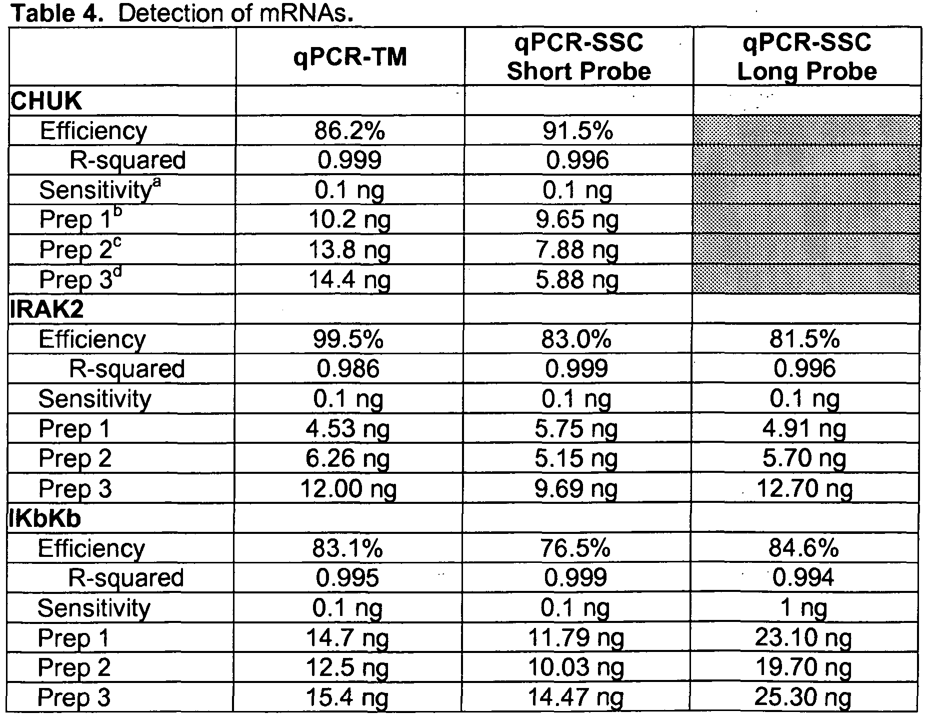

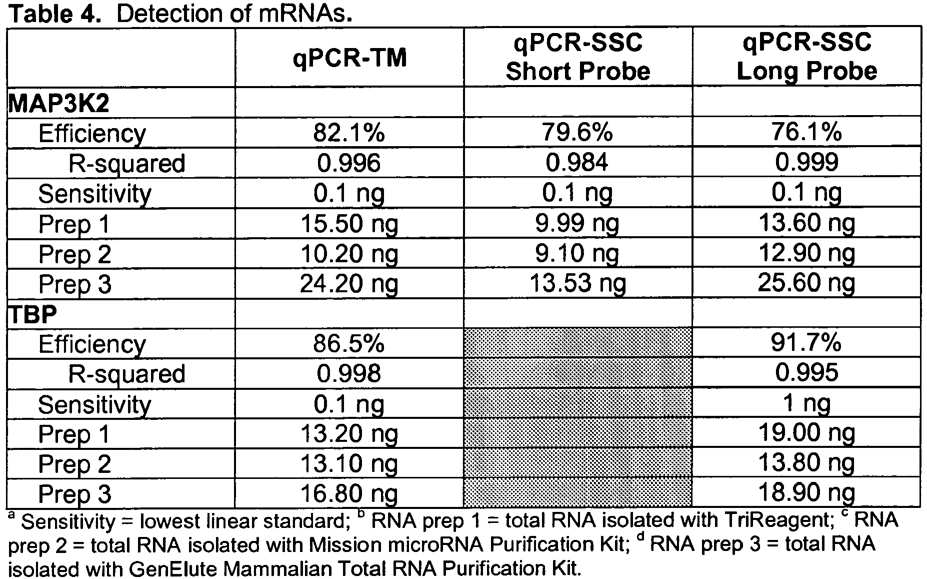

This experiment revealed that the qPCR-SSC detection system was able to detect mRNAs as well as a qPCR-TM system. Furthermore, the qPCR-SSC assay provides that advantage of requiring less hands-on time than the qPCR-TM assay.

### Example 5. Multiplexing with the real time qPCR-SSC system.

To determine whether more than one target could be detected simultaneously using the qPCR-SSC system, duplex reactions were also performed. qPCR-SSC assays were set up and performed as described in Example 4, using 5-fold serial dilutions of total RNA prepared from HeLa cells with TriReagent. Singleplex reactions were set up for each target, and separate duplex reactions were set up for each target (CHUK, IRAK2, IKbKb, and MAP3K2) in combination with TBP (whose probe contained a different fluorophore). Also included were control reactions with no template or 1 ng of DNA.

The efficiencies of these assays are presented in Table 5. Three of the four assays worked reasonably well in both singleplex and duplex reactions (i.e., the efficiencies were greater than about 80%), without optimizing either the primer concentration or the MgCl₂ levels. On the other hand, the CHUK and TBP assays worked well alone but not when combined in duplex reactions. These results indicate that more than one target can be detected simultaneously with real-time qPCR-SSC, but that optimization may be required.

**Table 5. Efficiencies of singleplex and duplex reactions.**

| | **GOI-s^{a}** | **GOI-d^{b}** | **TBP** |
|---|---|---|---|
| **CHUK** | 91.3% | 99.4% | 87.0% |
| **IKbKb** | 72.8% | 84.0% | 83.0% |
| **IRAK2** | 88.1% | 108.2% | 80.0% |
| **MAP3K2** | 81.6% | NL^{c} | NL |

| | | | |
|---|---|---|---|
| ^{a} GOI-s = gene of interest in singleplex reaction; ^{b} GOI-d = gene of interest in duplex reaction; ^{c} NL = not linear (i.e., R² for standard curves was >0.98). | | | |

### Example 6. Detection of single nucleotide polymorphisms (SNPs) using the real time qPCR-SSC system.

To determine whether single nucleotide polymorphisms in DNA could be detected in real time PCR using the structure-specific cleavage detection system, the protocol of the INVADER PLUS^{®} DNA SNP Kit assay was modified to allow detection during each cycle. The following targets were detected: TAS 2R16-1, TAS 2R16-2, TAS 2R16-3, and VKORC1.

Template DNA included: DNA purified from four cell lines (Wi38 cells, THP1 cells, HeLa cells, and HEK293 cells); DNA purified from two blood samples; DNA extracted from four blood samples; and purified DNA purchased from two suppliers. DNA was purified using a GenElute Mammalian Genomic DNA Miniprep Kit (G1N; Sigma-Aldrich, St. Louis, MO), and DNA was extracted using a RedExtract-n-Amp Blood Kit (XNAB; Sigma-Aldrich).

Each reaction contained 13.5 µL of water, 2 µL of 10X reaction buffer, 2 µL of primer/probe set, 0.5 µL of enzyme mix, and 2 µL of DNA (10 ng). Reactions containing of 0.1, 1.0, 10, and 100 ng were performed to generate standard curves and determine linear range. The reactions were thermocycled at 95°C, 2 min and 40 cycles (95°C, 30 sec, 63°C, 45 sec, 72°C, 1 min).

The PCR efficiencies were near 100% for TAS 2R16-2 and TAS 2R16-3, but were around 70-80% for TAS 2R16-1 and VKORC1. The VKORC1 amplicon was 505 bp in length, which is much longer than is recommended for qPCR and may explain its low efficiency of amplification. The purchased DNA was a mixture from several donors, and therefore, expected to contain both versions of all four alleles and give results similar to a heterozygous individual. Indeed, both were detected more or less equally. The delta Ct (ROX Ct - FAM Ct) was calculated for all of the samples, and those for TAS 2R16-1 are presented in Figure 6. It was reasoned that samples with delta Cts significantly lower the delta Cts of the purchased DNA samples were homozygous for the ROX allele, and that those with delta Cts significantly higher the delta Cts of the purchased DNA samples were homozygous for the FAM allele. These data reveal that SNPs may be detected in real time qPCR using a structure-specific cleavage detection system.

## Claims

1. A real time polymerase chain reaction process for detecting amplification of a target nucleic acid, a cycle of the process comprising:
(a) duplicating the target nucleic acid;
(b) hybridizing an unlabeled probe with a region of the amplified target nucleic acid, the probe comprising at least one portion that is complementary to the target nucleic acid and at least one portion that is not complementary to the target nucleic acid;
(c) cleaving the unlabeled probe to release a fragment; and
(d) detecting the released fragment using a universal detection module, the universal detection module comprising a region that is complementary to a portion of the released fragment, and the universal detection module comprising a hairpin structure, wherein the detection module generates a signal in the presence of the released fragment, such that a change in the signal relative to background indicates amplification of the target nucleic acid.

2. The process of claim 1, wherein duplication of the target nucleic acid is catalyzed by a thermostable DNA polymerase, optionally wherein the thermostable DNA polymerase is a wild-type enzyme or a modified enzyme obtained from the group of thermophilic organisms consisting of *Thermus aquaticus, Thermus flavus, Thermus thermophilus, Thermococcus litoralis,* and *Pyrococcus furiosus.*

3. The process of claim 1, wherein cleavage of the unlabeled probe is catalyzed by a thermostable endonuclease selected from the group consisting of a structure-specific endonuclease and a sequence-specific endonuclease, optionally wherein:
(i) the thermostable endonuclease is a wild-type enzyme or a modified enzyme obtained from the group of thermophilic organisms consisting of *Acidianus ambivalens, Acidianus brierlyi, Aeropyrum pernix, Archaeoglobus fulgidus, Archaeaglobus profundus, Archaeaglobus veneficus, Desulfurococcus amylolyticus, Desulfurococcus mobilis, Methanobacterium thermoautotrophicum, Methanococcus igneus, Methanococcus jannaschii, Methanopyrus kandleri, Pyrobaculum aerophilum, Pyrococcus furiosus, Pyrococcus horikoshii, Pyrococcus vvoesei, Pyrodictium brockii, Sulfolobus solfataricus, Thermus aquaticus, Thermus flavus, Thermus thermophilus, Thermococcus gorgonarius, Thermococcus litoralis,* and *Thermococcus zilligii;* and/or
(ii) the structure-specific endonuclease is selected from the group consisting of a flap endonuclease, a 5' flap endonuclease, a 3' flap endonuclease, a loop endonuclease, a hairpin endonuclease, and a DNA polymerase; and/or
(iii) the sequence-specific endonuclease is an enzyme that cleaves one strand of a double-stranded nucleic acid.

4. The process of claim 3, wherein hybridization between the complementary portion of the unlabeled probe and the target nucleic acid forms a duplex that abuts or overlaps with one end of a hybridized amplification primer or a probe, the non-complementary portion of the unlabeled probe remains single stranded forming a flap, and a flap endonuclease cleaves the unlabeled probe to release a fragment comprising the flap.

5. The process of claim 4, wherein the duplex region of the unlabeled probe overlaps with the 3' end of one of amplification primers, the flap is a 5' flap, and the flap endonuclease is a FEN-1 endonuclease.

6. The process of claim 1, wherein the detection module comprises a detection means; optionally wherein the detection means comprises a pair of fluorescence resonance energy transfer (FRET) interactive moieties.

7. The process of claim 6 , wherein hybridization between the released fragment and the detection module leads to cleavage of the detection module, the cleavage of the detection module separating the FRET interactive moieties and generating a change in the fluorescent signal; optionally wherein the cleavage of the detection module is catalyzed by
(i) a thermostable endonuclease selected from the group consisting of a structure-specific endonuclease and a sequence-specific endonuclease; or
(ii) the same thermostable endonuclease that cleaved the unlabeled probe.

8. The process of claim 1, wherein the change in the signal is an increase over background.

9. The process of claim 1, wherein the target nucleic acid is selected from the group consisting of an RNA molecule, a DNA molecule, and a hybrid RNA-DNA molecule; optionally wherein:
(i) the DNA is selected from the group consisting of complementary DNA (cDNA), nuclear DNA, organellar DNA, and genomic DNA; and/or
(ii) the RNA is selected from the group consisting of a messenger RNA, a mature small RNA, a mature microRNA, a precursor small RNA, and a precursor microRNA.

10. The process of claim 9, wherein the RNA is converted into cDNA by a reverse transcription reaction using one of the amplification primers; optionally wherein the reverse transcription reaction and the polymerase chain reaction occur in the same reaction mixture.

## Patentansprüche

1. Echtzeit-Polymerasekettenreaktionsverfahren zur Detektion der Amplifikation einer Zielnucleinsäure, wobei eine Runde des Verfahrens Folgendes umfasst:
(a) das Duplizieren der Zielnucleinsäure;
(b) das Hybridisieren einer unmarkierten Sonde mit einer Region der amplifizierten Zielnucleinsäure, wobei die Sonde zumindest einen Teil, der zu der Zielnucleinsäure komplementär ist, und zumindest einen Teil, der zu der Zielnucleinsäure nicht komplementär ist, umfasst;
(c) das Spalten der unmarkierten Sonde, um ein Fragment freizusetzen; und
(d) das Detektieren des freigesetzten Fragments unter Verwendung eines universellen Detektionsmoduls, wobei das universelle Detektionsmodul eine Region umfasst, die zu einem Teil des freigesetzten Fragments komplementär ist, und das universelle Detektionsmodul eine Haarnadelstruktur umfasst, worin das Detektionsmodul ein Signal in Gegenwart des freigesetzten Fragments erzeugt, sodass eine Veränderung des Signals relativ zum Hintergrund eine Amplifikation der Zielnucleinsäure anzeigt.

2. Verfahren nach Anspruch 1, worin die Duplizierung der Zielnucleinsäure von einer wärmebeständigen DNA-Polymerase katalysiert wird, worin gegebenenfalls die wärmebeständige DNA-Polymerase ein aus der aus *Thermus aquaticus, Thermus flavus, Thermus thermophilus, Thermococcus litoralis* und *Pyrococcus furiosus* bestehenden Gruppe von thermophilen Organismen ausgewähltes Wildtyp-Enzym oder modifiziertes Enzym ist.

3. Verfahren nach Anspruch 1, worin die Spaltung der unmarkierten Sonde durch eine aus der aus einer strukturspezifischen Endonuclease und einer sequenzspezifischen Endonuclease bestehenden Gruppe ausgewählte wärmebeständige Endonuclease katalysiert wird, worin gegebenenfalls:
(i) die wärmebeständige Endonuclease ein aus der aus *Acidianus ambivalens, Acidianus brierlyi, Aeropyrum pernix, Archaeoglobus fulgidus, Archaeoglobus profundus, Archaeoglobus veneficus, Desulfurococcus amylolyticus, Desulfurococcus mobilis, Methanobacterium thermoautotrophicum, Methanococcus igneus, Methanococcus jannaschii, Methanopyrus kandleri, Pyrobaculum aerophilum, Pyrococcus furiosus, Pyrococcus horikoshii, Pyrococcus woesei, Pyrodictium brockii, Sulfolobus solfataricus, Thermus flavus, Thermus thermophilus, Thermococcus gorgonarius, Thermococcus litoralis* und *Thermococcus zilligii* bestehenden Gruppe von thermophilen Organismen ausgewähltes Wildtyp-Enzym oder modifiziertes Enzym ist; und/oder
(ii) die strukturspezifische Endonuclease aus der aus einer Klappen-Endonuclease, einer 5'-Klappen-Endonuclease, einer 3'-Klappen-Endonuclease, einer Schleifenendonuclease, einer Haarnadelendonuclease und einer DNA-Polymerase bestehenden Gruppe ausgewählt ist; und/oder
(iii) die sequenzspezifische Endonuclease ein Enzym ist, das einen Strang einer doppelsträngigen Nucleinsäure spaltet.

4. Verfahren nach Anspruch 3, worin eine Hybridisierung zwischen dem komplementären Teil der unmarkierten Sonde und der Zieinucleinsäure ein Duplex bildet, das an ein Ende eines hybridisierten Amplifikationsprimers oder einer Sonde angrenzt oder sich damit überschneidet, wobei der nicht komplementäre Teil der unmarkierten Sonde einzelsträngig bleibt und eine Klappe bildet und eine Klappen-Endonuclease die unmarkierte Sonde spaltet, um ein Fragment freizusetzen, das die Klappe umfasst.

5. Verfahren nach Anspruch 4, worin die Duplexregion der unmarkierten Sonde sich mit dem 3'-Ende eines der Amplifikationsprimer überschneidet, die Klappe eine 5'-Klappe ist und die Klappen-Endonuclease eine FEN-1-Endonuclease ist.

6. Verfahren nach Anspruch 1, worin das Detektionsmodul ein Detektionsmittel umfasst, worin gegebenenfalls das Detektionsmittel ein Paar Fluoreszenzresonanzenergietransfer- (FRET-) Wechselwirkungsgruppierungen umfasst.

7. Verfahren nach Anspruch 6, worin eine Hybridisierung zwischen dem freigesetzten Fragment und dem Detektionsmodul zur Spaltung des Detektionsmoduls führt, wobei die Spaltung des Detektionsmodul die FRET-Wechselwirkungsgruppierungen trennt und eine Veränderung des fluoreszierenden Signals herbeiführt, worin gegebenenfalls die Spaltung des Detektionsmodul von Folgendem katalysiert wird:
(i) einer aus der aus einer strukturspezifischen Endonuclease und einer sequenzspezifischen Endonuclease bestehenden Gruppe ausgewählten wärmebeständigen Endonuclease; oder
(ii) der gleichen wärmebeständigen Endonuclease, die auch die unmarkierte Sonde gespalten hat.

8. Verfahren nach Anspruch 1, worin die Veränderung des Signals eine Erhöhung gegenüber dem Hintergrund ist.

9. Verfahren nach Anspruch 1, worin die Zielnucleinsäure aus der aus einem RNA-Molekül, einem DNA-Molekül und einem Hybrid-RNA-DNA-Molekül bestehenden Gruppe ausgewählt ist, worin gegebenenfalls:
(i) die DNA aus der aus komplementärer DNA (cDNA), nukleärer DNA, Organellen-DNA und genomischer DNA bestehenden Gruppe ausgewählt ist; und/oder
(ii) die RNA aus der aus einer Messenger-RNA, einer reifen kleinen RNA, einer reifen Mikro-RNA, einer kleinen Vorläufer-RNA und einer Vorläufer-Mikro-RNA bestehenden Gruppe ausgewählt ist.

10. Verfahren nach Anspruch 9, worin die RNA durch eine reverse Transkriptionsreaktion unter Verwendung eines der Amplifikationsprimer in cDNA übergeführt wird, worin gegebenenfalls die reverse Transkriptionsreaktion und die Polymerasekettenreaktion in demselben Reaktionsgemisch stattfinden.

## Revendications

1. Procédé de réaction en chaîne par polymérase en temps réel pour détecter l'amplification d'un acide nucléique cible, un cycle du procédé comprenant :
(a) dupliquer l'acide nucléique cible ;
(b) hybrider une sonde non marquée avec une région de l'acide nucléique cible amplifié, la sonde comprenant au moins une portion qui est complémentaire à l'acide nucléique cible et au moins une portion qui n'est pas complémentaire à l'acide nucléique cible ;
(c) cliver la sonde non marquée pour libérer un fragment ; et
(d) détecter le fragment libéré en utilisant un module de détection universel, le module de détection universel comprenant une région qui est complémentaire à une portion du fragment libéré, et le module de détection universel comprenant une structure en épingle à cheveux, où le module de détection produit un signal en présence du fragment libéré de sorte qu'un changement dans le signal relativement à l'arrière-plan indique l'amplification de l'acide nucléique cible.

2. Procédé selon la revendication 1, dans lequel la duplication de l'acide nucléique cible est catalysée par une polymérase d'ADN thermostable, en option où la polymérase d'ADN thermostable est une enzyme de type sauvage ou bien une enzyme modifiée obtenu du groupe d'organismes thermophiles consistant en *Thermus aquaticus, Thermus flavus, Thermus thermophilus, Thermococcus litoralis, et Pyrococcus furiosus.*

3. Procédé selon la revendication 1, dans lequel le clivage de la sonde non marquée est catalysé par une endonucléase thermostable sélectionnée dans le groupe consistant en une endonucléase structure-spécifique et une endonucléase séquence-spécifique, en option où :
(i) l'endonucléase thermostable est une enzyme de type sauvage ou bien une enzyme modifiée obtenue du groupe d'organismes thermophiles consistant en *Acidianus ambivalens, Acidianus brieriyi, Aeropyrum pernix, Archaeoglobus fulgidus, Archaeaglobus profundus, Archaeaglobus veneficus, Desulfurococcus amylolyticus, Desulfurococcus mobilis, Methanobacterium thermoautotrophicum, Methanococcus igneus, Methanococcus jannaschii, Methanopyrus kandleri, Pyrobaculum aerophilum, Pyrococcus furiosus, Pyrococcus horikoshii, Pyrococcus woesei, Pyrodictium brockii, Sulfolobus solfataricus, Thermus aquaticus, Thermus flavus, Thermus thermophilus, Thermococcus gorgonarius, Thermococcus litoralis,* et *Thermococcus zilligii*; et/ou
(ii) l'endonucléase structure-spécifique est sélectionnée dans le groupe consistant en une endonucléase rabat, une endonucléase 5'rabat, une endonucléase 3'rabat, une endonucléase en boucle, une endonucléase en épingle à cheveux et une polymérase d'ADN ; et/ou
(iii) l'endonucléase séquence-spécifique est une enzyme qui clive un brin d'un acide nucléique à brin double.

4. Procédé selon la revendication 3, dans lequel l'hybridation entre la portion complémentaire de la sonde non marquée et l'acide nucléique cible forme un duplex qui bute contre ou chevauche une extrémité d'une amorce d'amplification hybridée ou d'une sonde, la portion non-complémentaire de la sonde non marquée reste à brin unique formant un rabat, et une endonucléase rabat clive la sonde non marquée pour libérer un fragment comprenant le rabat.

5. Procédé selon la revendication 4, dans lequel la région duplex de la sonde non marquée se chevauche avec l'extrémité 3' d'une des amorces d'amplification, le rabat est un rabat 5', et l'endonucléase rabat est une endonucléase FEN-1.

6. Procédé selon la revendication 1, dans lequel le module de détection comprend un moyen de détection ; en option dans lequel le moyen de détection comprend une paire de fragments interactifs de transfert d'énergie de résonance de fluorescence (FRET).

7. Procédé selon la revendication 6, dans lequel l'hybridation entre le fragment libéré et le module de détection entraîne le clivage du module de détection, le clivage du module de détection séparant les fragments interactifs FRET et produisant un changement dans le signal fluorescent ; en option où le clivage du module de détection est catalysé par
(i) une endonucléase thermostable sélectionnée dans le groupe consistant en une endonucléase structure-spécifique et une endonucléase séquence-spécifique ; ou
(ii) la même endonucléase thermostable que a clivé la sonde non marquée.

8. Procédé selon la revendiation 1, dans lequel le changement dans le signal est un arrière-plan augmenté.

9. Procédé selon la revendication 1, dans lequel l'acide nucléique cible est sélectionné dans le groupe consistant en une molécule ARN, une molécule ADN, une molécule ARN-ADN hybride ; en option où :
(i) l'ADN est sélectionné dans le groupe consistant en un ADN complémentaire (ANDc), ADN nucléaire, ADN organellaire et ADN génomique ; et/ou
(ii) l'ARN est sélectionné dans le groupe consistant en un ARN messager, un petit ARN mature, un micro ARN mature, un petit ARN précurseur et un micro ARN précurseur.

10. Procédé selon la revendication 9, dans lequel l'ARN est converti en ADNc par une réaction de transcription inverse en utilisant une des amorces d'amplification ; en option où la réaction de transcription inverse et la réaction en chaîne par polymérase ont lieu dans le même mélange de réaction.


### SEQUENCE LISTING

<110> SIGMA-ALDRICH CO. KRAEDER, Carol WARD, Brian
<120> REAL TIME POLYMERASE CHAIN REACTION PROCESS USING A UNIVERSAL DETECTION SYSTEM
<130> 47497-TBA
<150> US 61/073,198
   <151> 2008-06-17
<160> 6
<170> PatentIn version 3.5
<210> 1
   <211> 23
   <212> RNA
   <213> Artificial Sequence
<220>
   <223> HOMO SAPIENS
<400> 1
   uaaagugcuu auagugcagg uag 23
<210> 2
   <211> 23
   <212> RNA
   <213> Artificial Sequence
<220>
   <223> HOMO SAPIENS
<400> 2
   caaagugcuc auagugcagg uag 23
<210> 3
   <211> 21
   <212> RNA
   <213> Artificial Sequence
<220>
   <223> HOMO SAPIENS
<400> 3
   uaaagugcug acagugcaga u 21
<210> 4
   <211> 23
   <212> RNA
   <213> Artificial Sequence
<220>
   <223> HOMO SAPIENS
<400> 4
   caaagugcuu auagugcagg uag 23
<210> 5
   <211> 23
   <212> RNA
   <213> Artificial Sequence
<220>
   <223> HOMO SAPIENS
<400> 5
   uaaagugcuc auagugcagg uag 23
<210> 6
   <211> 23
   <212> RNA
   <213> Artificial Sequence
<220>
   <223> HOMO SAPIENS
<400> 6
   uaaagugcuu auagugcagg uaa 23